# EUROPEAN PATENT SPECIFICATION

(11) **EP 0 660 704 B1**
(45) Date of publication and mention of the grant of the patent: **17.09.1997**
(21) Application number: 92912048.3
(22) Date of filing: 16.06.1992
(51) Int. Cl.: A61K 9/02, A61J 3/08

(54) **A PROCESS FOR PRODUCING SUPPOSITORIES BY COMPRESSION AND SUPPOSITORIES OBTAINED BY THE PROCESS**
VERFAHREN ZUR HERSTELLUNG VON SUPPOSITORIEN DURCH KOMPRESSION UND DURCH DIESES VERFAHREN ERHALTENE SUPPOSITORIEN
PROCEDE DE PREPARATION DE SUPPOSITOIRES PAR COMPRESSION ET SUPPOSITOIRES OBTENUS SELON CE PROCEDE

(30) Priority: 17.06.1991 DK 1165/91
(43) Date of publication of application: 05.07.1995
(73) Proprietor: FARMACEUTISK LABORATORIUM FERRING A/S, DK-2720 Vanlöse (DK)
(72) Inventor: HALSKOV, Sören, DK-2830 Virum (DK)
(74) Representative: Christiansen, Ejvind
(86) International application number: DK9200187
(87) International publication number: WO9222283

(56) References cited:
- EP-A- 0 111 137
- DE-A- 2 248 777

## Description

### Field of the invention

The present invention concerns a special process for producing suppositories by compression. The invention moreover concerns the suppositories obtained by the process which have a considerably higher percentual content of active drug than ordinary suppositories.

### Background of the invention

As will be known, suppositories are drugs intended for insertion into the rectum. They contain the active drug in a dosed amount and are produced by pressing, moulding or compression. They can also be produced in the form of capsules for controlled release of the active substance.

Ordinarily suppositories are produced by moulding, the produced mass being melted using the least possible amount of heat, and then the liquid mass is poured into moulds having the desired nominal capacity.

The suppositories produced by moulding are oblong and smooth, and they have a uniform appearance. Melting is intended to provide a uniform distribution of the drug in the base mass, which, however, can be difficult to obtain because of sedimentation during hardening.

However, traditional moulding is a time-consuming and slow process which involves considerable costs. Moulded suppositories also have the drawback that too strong heating of certain suppository base masses result in unstable modifications with a considerably reduced solidification point.

It is well-known to use polyethylene glycols having average molecular weights of 4000-6000 or above as the main component in the base mass for suppositories produced by traditional melting and moulding. Thus, the DE-A-2.248.777 describes melt-moulded indomethacin suppositories whose base mass contains such polyethylene glycols. The content of indomethacin in the examples is about 3.5% by weight and the amount of polyethylene glycols is above80 %.

It has previously been attempted to produce suppositories by compression or pressing, i.e. by traditional tabletting methods. However, these suppositories tend to form irregular rough surfaces, which makes them unpleasant to use for the patient. Moreover, in such a production method it has been found impossible to incorporate the drug in so high doses as is often desirable owing to the prescribed treatment.

Thus, the EP-A2-111 137 describes suppositories containing the drug indomethacin in a base consisting of polyethylene glycol having an average molecular weight of up to 35,000. It is stated that the content of the active drug may be up to 50% by weight, but preferably the content is 2-40% by weight and in particular 2-26% by weight. All the examples in the publication concern rectal tablets having a content of indomethacin of 2.8 - 5.8% by weight and rectal capsules having a content of indomethacin of 5.25 - 10.5% by weight, i.e. rather low concentrations. Further, the suppositories thus known contain quite high amounts of polyethylene glycols, typically 1600-1730 mg per unit, which is a drawback, because it has been found that a content of polyethylene glycols in suppositories of 1 - 1.5 g per unit may cause bowel disorders.

### Summary of the invention

An object of the invention is to provide a process enabling the production of suppositories having a high content of active drug, which are partly more convenient to store and are partly more convenient to use and easier and cheaper to produce than traditional melt-moulded suppositories.

This is achieved by the process of the invention which is characterized by producing a suppository mixture granulate containing (a) 50-75% active drug, optionally including microcrystalline cellulose and/or other additives common in the production of drugs, and (b) 2-5% talc, magnesium stearate and/or polyvinyl pyrrolidone, the balance (c) consisting of a polyethylene glycol having an average molecular weight of at least 4000, and compressing the produced mass to suppositories by a method known per se for the production of tablets.

It was surprisingly found that such a composition could easily be compressed to suppositories by an ordinary tabletting method. The use of the polyethylene glycol in the base mass results in suppositories having a uniform appearance and having an extremely smooth and regular surface, which is moreover sufficiently slippery for the suppository to be inserted without difficulty. It is moreover possible in such suppositories to incorporate up to 75% by weight of active drug, which is far above normal.

### Detailed description of the invention

Polyethylene glycols, more particularly mixtures of condensation polymers of ethylene oxide and water, are also called "macrogols". Macrogols having average molecular weights of 200-700 are liquids, while macrogols having average molecular weights above 1000 vary in consistence from soft oily substances to hard wax-like solid substances. The average molecular weight is stated as a number after the name. "Macrogol 6000", which it is preferred to use according to the invention, thus has an average molecular weight of about 6000.

The macrogols have the general formula

H(CH₂OCH₂)ₙOH

where n is greater than or equal to 4. In "Macrogol 6000" n has an average value of between 158 and 204. It is a white or cream-coloured solid wax-like substance which is in the form of a powder or flakes. The melting point is 56-63°C, i.e. considerably above the body temperature. Mixtures of various polyethylene glycols having melting points above the body temperature have frequently been used as a base in suppositories from which the drug is released by dissolution.

Since the melting points of the macrogols increase with increasing average molecular weight, macrogols having high average molecular weights, such as "Macrogol 6000", have not previously been a natural choice as a suppository base material.

In addition to a polyethylene glycol or a mixture of polyethylene glycols having an average molecular weight of at least 4000, preferably "Macrogol 6000", the suppositories produced according to the invention may contain microcrystalline cellulose and/or other additives common in the production of drugs. These additives and the active drug together amount to 50-75% based on the gross weight of the suppositories. Further, the suppositories contain one or more of the substances talc, magnesium stearate and polyvinyl pyrrolidone in an amount of 2-5% by weight, the balance up to 100% consisting of the polyethylene glycol.

Mixing a granulate of the drug with "Macrogol 6000" and compressing the mixture to suppositories by a method which is known per se for the production of tablets for oral administration provide the following advantages:
1) The suppositories do not melt under normal temperature conditions, but only at about 60°C.
2) The suppositories are cheaper to produce.
3) The drug can be incorporated in very high concentrations and is considerably easier to dose uniformly.
4) The product is more convenient and more pleasant to use for the patient.
5) Avoidance of drug decomposition during the heating which is necessary in traditional moulding of suppositories.
6) It is easier to obtain and maintain a homogeneous mixture, and the risk of sedimentation is eliminated.

All active drugs which lend themselves for rectal administration may be incorporated in the suppositories produced according to the invention. One of the interesting drugs in this respect is 5-aminosalicylic acid (5-ASA), which is used particularly for the treatment of colitis ulcerosa and Crohn's disease, but which has moreover been found to be of interest for the production of suppositories for treatment of hemorrhoids. Suppositories containing 5-aminosalicylic acid are known e.g. from the EP patent application 83 775, these suppositories being produced by moulding and containing max. 500 mg of 5-aminosalicylic acid per dose unit. The suppositories of the invention may contain considerably larger amounts of 5-ASA, which is absolutely an advantage for the patient.

An example of the many active drugs which may be used is steroids for various applications.

The suppositories are typically compressed to symmetrical units having an approximately elliptic longitudinal section, i.e. the two ends are uniform (in contrast to the ordinary "torpedo-shape" where one end is pointed while the other is blunt).

The following example illustrates the invention:

### EXAMPLE

A suppository base mass for the production of 1000 suppositories consists of the following ingredients:

| | |
|---|---|
| "Macrogol 6000" | 572 g |
| Active drug and microcrystalline cellulose | |
| | 1000 g |
| Magnesium stearate | 4 g |
| Talc | 4 g |
| Polyvinyl pyrrolidone + ethanol (1:19) | q.s. |

A granulate is made from the microcrystalline cellulose, the active drug, e.g. 5-aminosalicylic acid (5-ASA) and the mixture of polyvinyl pyrrolidone and ethanol.

The resulting granulate is mixed with "Macrogol 6000", and then magnesium stearate and talc are added.

The final granulate is compressed to suppositories in a tabletting machine in a manner known per se.

## Claims

1. A process for producing suppositories by compression,
**characterized** by producing a suppository mixture granulate containing (a) 50-75% active drug, optionally including microcrystalline cellulose and/or other additives common in the production of drugs, and (b) 2-5% talc, magnesium stearate and/or polyvinyl pyrrolidone, the balance (c) consisting of a polyethylene glycol having an average molecular weight of at least 4000, and compressing the produced mass to suppositories by a method known per se for the production of tablets.

2. A process according to claim 1,
**characterized** in that the polyethylene glycol used has an average molecular weight of 6000.

3. A process according to claim 1,
**characterized** in that the polyethylene glycol used is selected from "Macrogol 4000", "Macrogol 6000" and mixtures thereof.

4. Suppositories,
**characterized** in that they are produced by a process according to any of claims 1-3.

5. Suppositories according to claim 4,
**characterized** in that the polyethylene glycol used is "Macrogol 6000".

6. Suppositories according to claim 4 or 5,
**characterized** in that they contain 5-aminosalicylic acid (5-ASA) as an active drug in an amount of up to 75% of the gross weight of the product.

7. Suppositories according to claim 4 or 5,
**characterized** in that they contain a steroid as an active drug.

## Patentansprüche

1. Verfahren zur Herstellung von Zäpfchen mittels Pressen, dadurch gekennzeichnet, daß eine Zäpfchengranulatmischung, enthaltend (a) 50-75% eines aktiven Wirkstoffes, gegebenenfalls einschließlich mikrokristalliner Zellulose und/oder anderer Additive, die bei der Herstellung von Arzneimitteln üblich sind, und (b) 2 bis 5% Talkum, Magnesiumstearat und/oder Polyvinylpyrrolidon, der Rest (c) bestehend aus Polyethylenglykol mit einem mittleren Molekulargewicht von mindestens 4000, und Pressen der hergestellten Masse zu Zäpfchen durch ein Verfahren wie es an sich für die Herstellung von Tabletten bekannt ist.

2. Verfahren gemäß Anspruch 1, dadurch gekennzeichnet, daß das Polyethylenglykol ein mittleres Molekulargewicht von 6000 aufweist.

3. Verfahren gemäß Anspruch 1, dadurch gekennzeichnet, daß das Polyethylenglykol, welches verwendet wird, ausgewählt ist aus "Macrogol 4000", "Macrogol 6000" und Mischungen hiervon.

4. Zäpfchen, dadurch gekennzeichnet, daß sie mit einem Verfahren gemäß einem der Ansprüchen 1 bis 3 hergestellt sind.

5. Zäpfchen nach Anspruch 4, dadurch gekennzeichnet, daß als Polyethylenglycol "Macrogol 6000" verwendet wird.

6. Zäpfchen nach Anspruch 4 oder 5, dadurch gekennzeichnet, daß sie 5-Aminosalicylsäure (5-ASA) als einen aktiven Wirkstoff in einer Menge bis zu 75% des Gesamtgewichts des Produkts enthalten.

7. Zäpfchen nach Anspruch 4 oder 5, dadurch gekennzeichnet, daß sie ein Steroid als einen aktiven Wirkstoff enthalten.

## Revendications

1. Procédé de production de suppositoires par compression, caractérisé par la production d'un granulé en mélange pour suppositoires contenant (a) 50-75% d'un médicament actif, comprenant éventuellement de la cellulose microcristalline et/ou d'autres additifs usuels dans la production de médicaments, et (b) 2-5% de talc, de stéarate de magnésium et/ou de polyvinylpyrrolidone, le complément (c) étant constitué d'un polyéthylèneglycol ayant un poids moléculaire moyen d'au moins 4 000, et la compression de la masse produite en suppositoires par une méthode connue en soi pour la production de comprimés.

2. Procédé selon la revendication 1, caractérisé en ce que le polyéthylèneglycol utilisé a un poids moléculaire moyen de 6 000.

3. Procédé selon la revendication 1, caractérisé en ce que le polyéthylèneglycol utilisé est choisi parmi le "Macrogol 4000", le "Macrogol 6000" et des mélanges de ceux-ci.

4. Suppositoires, caractérisés en ce qu'ils sont produits par un procédé selon l'une des revendications 1-3.

5. Suppositoires selon la revendication 4, caractérisés en ce que le polyéthylèneglycol utilisé est le "Macrogol 6000".

6. Suppositoires selon la revendication 4 ou 5, caractérisés en ce qu'ils contiennent l'acide 5-aminosalicylique (5-ASA) à titre de médicament actif en une quantité allant jusqu'à 75% du poids brut du produit.

7. Suppositoires selon la revendication 4 ou 5, caractérisés en ce qu'ils contiennent un stéroïde à titre de médicament actif.
